# EUROPEAN PATENT APPLICATION

(11) **EP 2 387 907 A1**
(43) Date of publication of application: **23.11.2011**
(21) Application number: 10005236.4
(22) Date of filing: 19.05.2010
(51) Int. Cl.: A45D 34/02, A45D 34/04, A47L 15/00, A61L 2/14, H05H 1/24, H05H 1/30, H05H 1/34, A61N 1/44, B66B 31/02

(54) **Appliance, particularly kitchen appliance or laboratory table**

(71) Applicant: Max-Planck-Gesellschaft zur Förderung der Wissenschaften e.V., 80539 München (DE)
(72) Inventor: The designation of the inventor has not yet been filed
(74) Representative: Gleiss & Grosse

(57) **Abstract**

The invention relates to an appliance (18), particularly kitchen appliance or laboratory table, for at least partially disinfecting/sterilising a contaminated surface (21), wherein the appliance (18) comprises an integrated plasma source for at least partially disinfecting/sterilising the surface by generating a non-thermal atmospheric plasma on the surface thereby reducing the concentration of pathogenic germs on the surface.

## Description

### Field of the invention

The invention relates to an appliance, particularly a kitchen appliance or a laboratory table, for at least partially sterilizing a contaminated surface.

### Background of the invention

The use of non-equilibrium plasmas (often referred to as non-thermal plasmas or low-temperature plasmas) for the in vivo sterilization of wounds is disclosed, for example, in U.S. patent US 7,683,342 B2. However, the plasma source disclosed in this patent is not suitable for the regular sterilization of surfaces of appliances, e.g. kitchen appliances or laboratory tables, under normal operating conditions of the appliance, i.e. during daily use of the appliance.

### Summary of the invention

It is therefore an object of the invention to improve the disinfection of surfaces of appliances, particularly kitchen appliances or laboratory tables.

This problem is solved by the idea to integrate a plasma source into an appliance, e.g. kitchen appliance or a laboratory table, wherein the integrated plasma source at least partially disinfects the surface by generating a non-thermal plasma on the surface thereby reducing the concentration of pathogenic germs on the surface.

In a preferred embodiment of the invention, the surface to be disinfected is a surface of the appliance which is contaminated during use of the appliance. For example, kitchen appliances, e.g. work benches, generally comprise work plates or cutting boards, which are contaminated during the preparation of food. In other words, the surface to be sterilized is preferably a part of the appliance which also includes the plasma source.

However, it is alternatively possible that the surface to be sterilized is separated from the appliance including the plasma source. For example, the invention also encompasses a deodorant device for deodorizing a body surface, particularly in the form of a roll-on applicator or a spray device. In this embodiment, the plasma source is integrated into the deodorant device while the surface to be sterilized is a body surface which is separated from the deodorant device.

In a preferred embodiment of the invention, the plasma source is a surface micro-discharge plasma source comprising several electrodes, wherein the surface micro-discharge plasma source generates micro-discharges on the surface of the plasma source. The basic principles of high-pressure plasma micro-discharges are explained, for example, in Hippler/Kersten/Schmidt/Schoenbach: "Low temperature plasmas", Second Edition, Wiley Publishing House, Chapter 17. Therefore, reference is made to the afore-mentioned publication with regard to the basic principles of surface micro-discharge plasma sources, so that the afore-mentioned publication is incorporated by reference herein. However, it should be briefly mentioned that the surface micro-discharge plasma source comprises several electrodes which are spaced apart.

It should further be mentioned that there can be a uniform distance between the adjacent electrodes of different polarity. However, it is alternatively possible that there is spatially variable distance between the adjacent electrodes of different polarity.

Further, it should be noted that the surface micro-discharge plasma source is preferably embedded into the surface of the appliance, so that the non-thermal plasma is generated on top of the surface of the appliance. For example, the plasma source can be embedded in the surface of a work plate of a kitchen table so that the low-temperature plasma is generated on the surface of the work plate of the kitchen table thereby at least partially sterilizing the surface of the work plate.

It should further be noted that the embedded plasma source is preferably embedded in such a way that it is substantially flush with the surface of the appliance. Therefore, the plasma source preferably comprises a substantially plane surface which is flush with the plane surface of the appliance, e.g. a work plate of a kitchen table.

However, the invention is not restricted to appliances comprising a plane plasma source. It is also possible that the electrodes of the surface micro-discharge plasma source have a shape which resembles the shape of the surface of the appliance. For example, the plasma source can be integrated in a curved surface of the appliance so that the invention does not restrict the freedom of design of the appliance.

Further, there is variety of different arrangements of the electrodes of the surface micro-discharge plasma source.

In one embodiment of the invention, the electrodes of the surface micro-discharge plasma source are arranged in the same plane. For example, the electrodes can be finger-shaped intertwining each other from opposite directions. In another embodiment, the electrodes are spiral-shaped intertwining each other. Further, the electrodes can comprise interlocking branches or kinks.

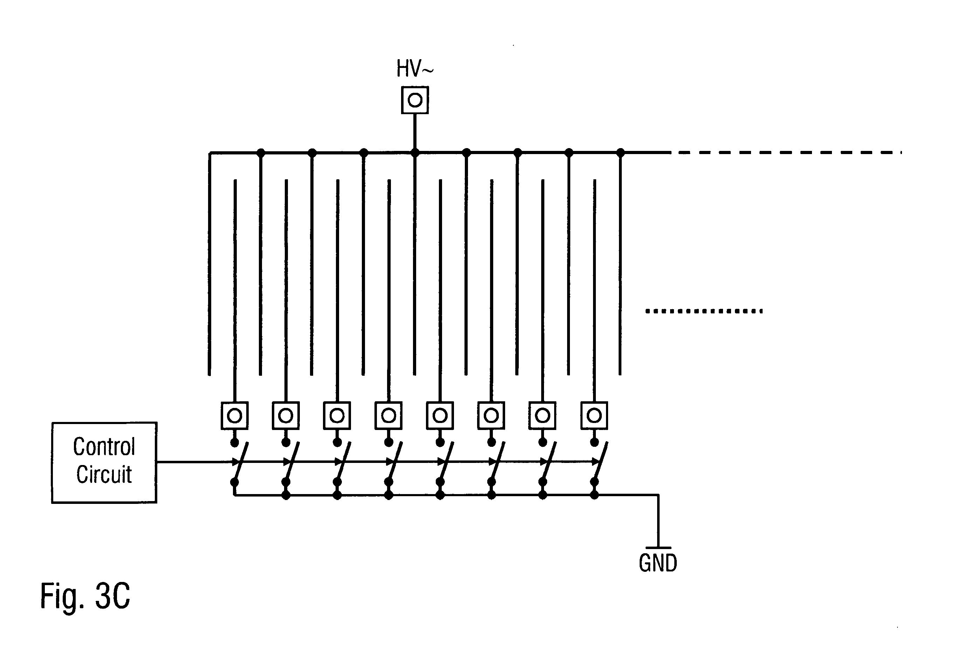

In another embodiment of the invention, the electrodes are accessed sequentially by switching of the grounded part with a "cycling frequency" f_{c}, thus enabling a propagating plasma source across the device. For instance, a "switched self sterilizing surface device" (100×50cm²) with parallel electrodes (see Figure 3C) separated by 5mm operated with a cycling frequency of 1Hz would have plasma production along a strip of length 50cm for a time tₚ=10ms. For a HV frequency f_{Hv}=2kHz this would imply f_{HV}·tₚ 20 bursts of surface micro discharges, enough to start the ion-molecule reaction chain. The plasma afterglow lasts for more than 1s, so that the next cycle would enhance the local plasma chemistry. Such a device (100×50cm²) could be operated with a power of 25W.

In another embodiment of the invention, the electrodes of the micro-discharge plasma source are not arranged in the same plane but in separate adjacent electrode layers, wherein each of the electrode layers is preferably planar and the separate electrode layers are preferably arranged coplanar relative to each other.

Moreover, it should be noted that the electrode arrangement of the plasma source is freely scaleable.

Further, it should be mentioned that the appliance according to the invention is preferably water-proof, dust-proof, air-born particles proof and/or easy to clean. This is particularly advantageous in case of a kitchen table comprising an integrated plasma source for disinfecting the work-plate of the kitchen table.

Moreover, the surface of the appliance comprising the integrated plasma source preferably consists of a corrosion resistant material, particularly ceramics, glass or glass-ceramics.

It has already been mentioned that the appliance according to the invention can be a work plate, particularly on a kitchen table or on a laboratory table, or a cutting board for cutting objects, particularly food stuffs.

However, the plasma source according to the invention can alternatively be integrated into a handle, particularly a door handle, wherein the integrated plasma source sterilizes the surface of the handle.

In another embodiment of the invention, the appliance is a bathroom equipment, particularly a toilet seat, comprising an integrated plasma source for sterilizing the surface of the bathroom equipment.

Further, it has already been mentioned that the invention also encompasses a deodorant device for deodorizing a body surface particularly in the form of a roll-on applicator or a spray device. In this embodiment, the deodorant device comprises an integrated plasma source applying a non-thermal plasma to the body surface which is to be deodorized.

Another application of the invention is the sterilization of a moving handrail of an escalator or a moving walkway. In this embodiment, the plasma source can be arranged stationary close to the surface of the handrail, so that the non-thermal plasma generated by the plasma source at least partially disinfects/sterilizes the surface of the handrail. Alternatively, the plasma source can also be integrated into the moving handrail, so that the plasma source moves with the handrail.

Further, the invention also encompasses an appliance in the form of gym equipment, particularly in the form of a bench or a seat of a training machine. In this embodiment, the plasma source is integrated into the gym equipment thereby sterilizing the bacteria produced by the sweat.

It should also be noted that the power supply of the integrated plasma source can be provided wireless by an integrated battery which allows a mobile use of the device. Alternatively, the power supply of the plasma source can be provided by connection to the general mains.

Further, the invention encompasses also a battery operated device for reducing itching caused by insect bite, particularly in the form of a stick comprising the plasma source. In this embodiment, the battery operated device applies a non-thermal plasma to the skin surface at an insect bite thereby reducing itching.

Further, the invention is also suitable for protection against athletes' foot and other fungal deceases, particularly in damp environments, particularly swimming pools and saunas.

Moreover, the invention is suitable for the disinfection of baby bottles, pacifiers, toys, dentures, tooth brushes, razors, shavers, combs or hair brushes.

Another possible application of the invention is the use of a non-thermal plasma in a dishwasher or a dryer for sterilizing the dishes in the dishwasher or dryer.

Further, the concept of the invention can be applied in devices for disinfection of medical equipment or in the food industry for disinfecting objects.

In another embodiment, the appliance according to the invention comprises a conveyor belt, wherein the plasma source is arranged in the vicinity of the conveyor belt so that the plasma sterilizes objects conveyed on the conveyor belt. For example, the plasma source can be arranged beneath the conveyor belt so that the plasma is applied through the belt, which therefore has to be permeable for the plasma. Alternatively, the plasma source can be arranged stationary above or in the vicinity of the conveyor belt so that the plasma generated by the plasma source reaches the objects on the conveyor belt. Further, it is alternatively possible to integrate the plasma source into the conveyor belt.

It has already been mentioned that the invention also encompasses a deodorant device for deodorizing a body surface by applying a non-thermal plasma. In one embodiment, the deodorant device of the invention resembles the design of conventional deodorant devices comprising a rotatable ball. In this embodiment, the plasma source can be integrated into the rotatable ball, wherein the plasma source generates the plasma on the surface of the rotatable ball. Alternatively, the plasma source can be arranged stationary within the housing of the deodorant device but outside the rotatable ball.

In a preferred embodiment of the deodorant device according to the invention, the deodorant device additionally comprises an applicator for applying a deodorant to the skin surface wherein the agent applied to the skin surface interacts with the non-thermal plasma thereby improving the disinfecting/sterilizing effect of the non-thermal plasma. In other words, the non-thermal plasma applied by the plasma source and the chemical agent applied by the applicator interact with each other so that the disinfecting/sterilizing effect is enhanced by the interaction between the chemical agent and the non-thermal plasma.

In another embodiment, the deodorant device does not comprise any moveable parts and relies solely on the disinfecting/sterilizing effect of the plasma.

It should also be noted that the term "pathogenic germs" as used in this description encompasses bacteria, spores, viruses, fungi, prions, micro organisms and bio-films comprising any of the afore-mentioned pathogenic germs.

The invention and its particular features and advantages will become more apparent from the following detailed description considered with reference to the accompanying drawings.

### Brief description of the drawings

- Figure 1: shows an exploded perspective view of a plasma source according to the invention, which can be integrated into an appliance, e.g. a kitchen table.
- Figure 2: shows a perspective view of the plasma source according to Figure 1.
- Figures 3A-3I: show different embodiments and views of a plasma source according to the invention, including the "switched self sterilizing surface" in Figure 3C, wherein switches are closed and opened sequentially at a rate f_{c}/n, with n being the number of grounded electrodes.
- Figure 4: shows a perspective view of a kitchen block comprising a self-sterilizing work plate.
- Figure 5: shows a perspective view of a laboratory table comprising a self-sterilizing work plate.
- Figure 6: shows a perspective view of a toilet seat comprising an integrated plasma source for sterilizing the toilet seat.
- Figure 7A: shows a schematic view of an escalator comprising a plasma source for sterilizing the moving handrail of the escalator.
- Figure 7B: shows a modification of the embodiment of Figure 7A wherein the plasma source is integrated into the moving handrail.
- Figure 8: shows a schematic view of the deodorant device comprising an integrated plasma source.
- Figure 9: shows a modification of the embodiment of Figure 8 additionally comprising nozzles for applying a chemical agent onto the body surface.
- Figure 10: is a modification of the embodiment of Figure 8 comprising a rotatable ball wherein the plasma source is integrated into the rotatable ball.
- Figure 11: shows a perspective view of another example of a deodorant device comprising an integrated plasma source.
- Figure 12: shows a schematic view of a washing machine comprising an integrated plasma source.
- Figure 13: shows a simplified side view of a conveyor comprising a plasma source for sterilizing objects on the conveyor.
- Figure 14: is a diagram showing the switching of the plasma source in the appliance according to the invention.

### Detailed description of the drawings

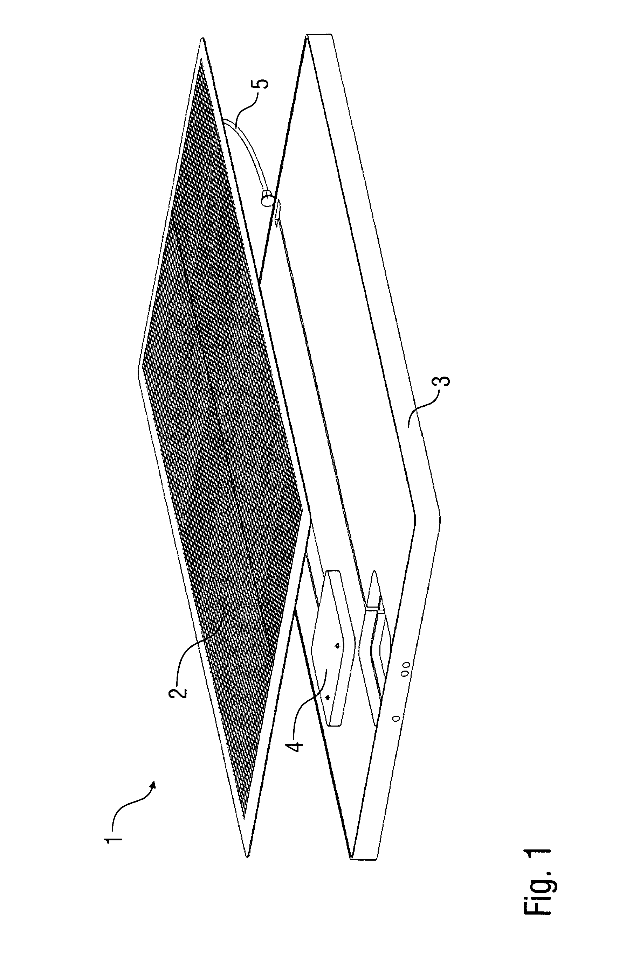

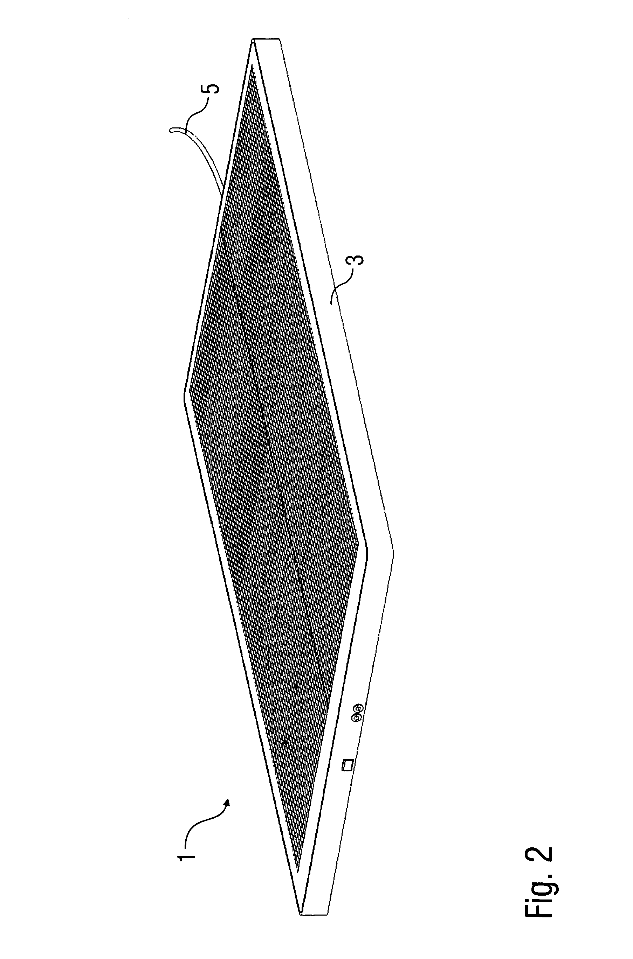

Figure 1 and 2 illustrate an embodiment of a plasma source 1 which can be integrated into an appliance, e.g. a work plate of a kitchen table, which will be described in more detail later.

The plasma source 1 comprises a flat and planar electrode arrangement 2, a housing 3, a driver circuit 4 for driving the electrode arrangement 2 and a connection cable 5 for connecting the plasma source 1 to mains.

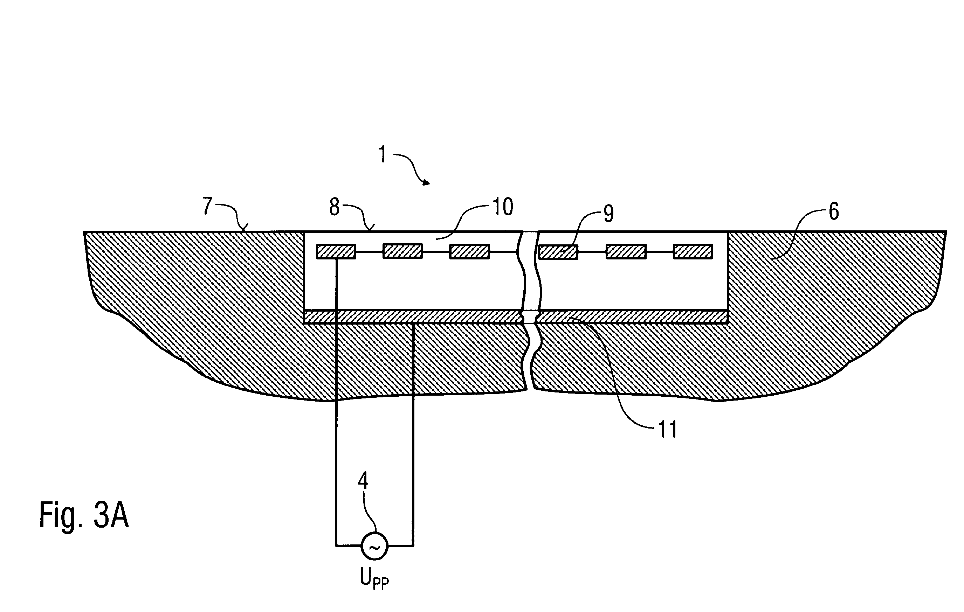

Figure 3A shows a simplified cross section of a first embodiment of the electrode arrangement 2 of the plasma source 1.

Firstly, it should be noted that the plasma source 1 is integrated into an open cavity of an appliance 6 so that the appliance 6 and the plasma source 1 comprise surfaces 7, 8 which are flush so that the surface 8 of the plasma source 1 constitutes a part of the surface 7 of the appliance 6.

Further, it should be noted that the electrode arrangement 2 of the plasma source comprises several grid-shaped electrodes 9 which are interconnected with each other and embedded into a dielectric layer 10. Further, the electrode arrangement 2 comprises a common back electrode 11 at the under side of the dielectric layer 10.

During operation, the driver circuit 4 applies a AC voltage U to the electrodes 9, 11 so that surface micro-discharges are triggered on the surface 8 of the plasma source 1 as explained in the above-mentioned book titled "Low temperature plasmas".

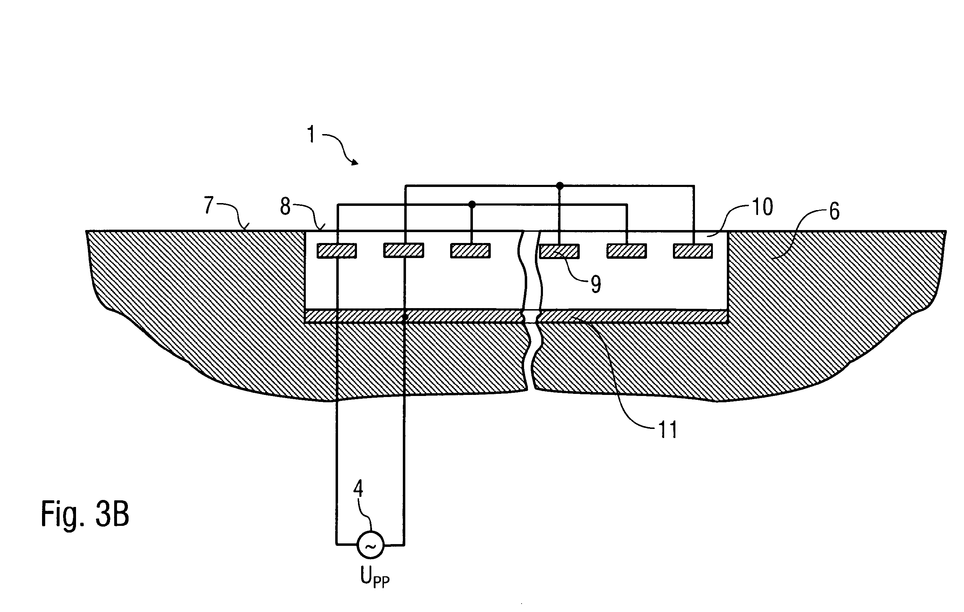

Figure 3B shows a modification of the plasma source 1 according to Figure 3A, wherein this embodiment corresponds to the previous embodiment to a large extent. Therefore, reference is made to the above description of the embodiment according to Figure 3A and the same reference numerals are used for corresponding parts and details.

One characteristic of this embodiment is the electrical connection of the upper electrodes 9 which are alternatively connected to the different poles of the driver circuit 4. Figure 3C shows another modification of the embodiment of Figure 3A, wherein this embodiment corresponds to the previous embodiment to a large extent. The difference lies in the mode of operation, the switching of the discharge from one segment to the next. This technique makes use of the plasma afterglow and allows production of large self sterilizing surfaces with low energy requirements.

Therefore, reference is made to the above description with regard to Figure 3A and the same reference numerals are used for corresponding parts and details.

One characteristic of this embodiment is that the back electrode 11 is missing.

It should further be noted that it is alternatively possible to switch the high voltage lines, as well. Further, it is possible to switch all electrode pairs successively pair by pair.

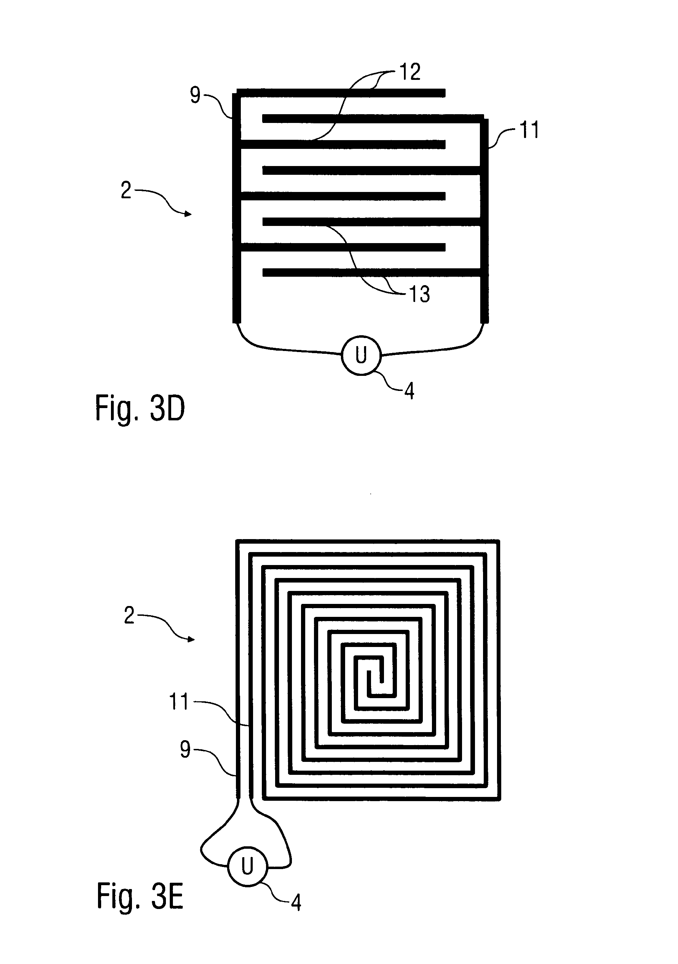

Figure 3D shows an alternative design of the electrode arrangement 2, wherein the electrodes 9 and 11 each comprise electrode fingers 12, 13 intertwining each other. In this embodiment, the electrodes 9, 11 along with their electrode fingers 12, 13 are arranged in the same plane.

Figure 3E shows another design of the electrode arrangement 2 wherein the electrodes 9, 11 are spiral-shaped intertwining each other.

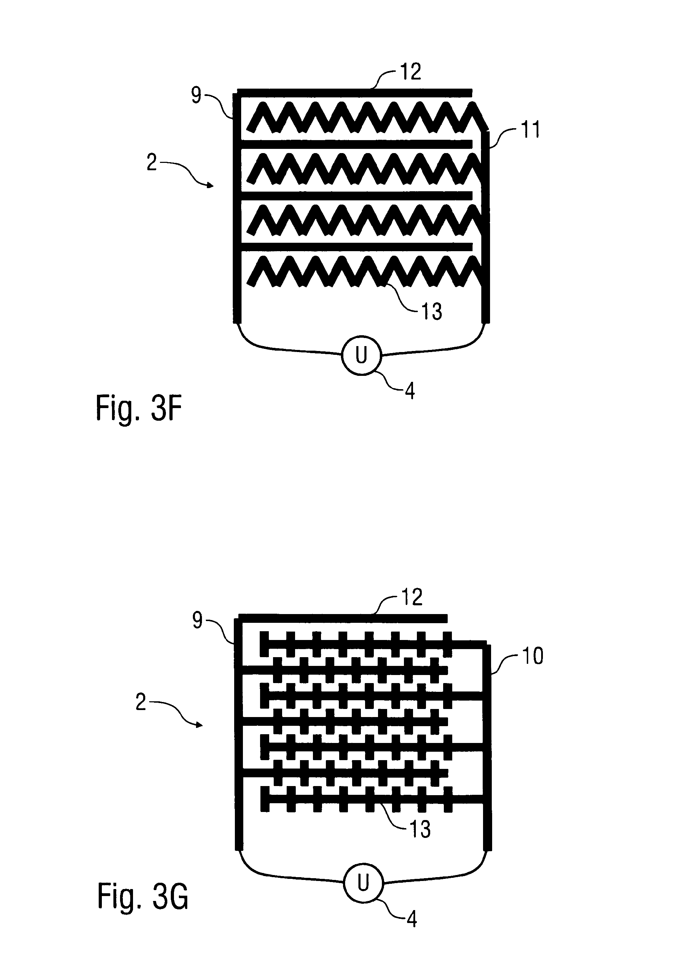

Figure 3F shows a modification of the electrode arrangement 2 of Figure 3D, wherein this embodiment corresponds to the embodiment of Figure 3D to a large extent. Therefore, reference is made to the above description with regard to Figure 3D and the same reference numerals are used for corresponding parts and details.

One characteristic of this embodiment is that the electrode fingers 13 of the electrode 11 are staggered.

Figure 3G shows another modification of the electrode arrangement, wherein the electrode fingers 13 of the electrode 11 comprises interlocking branches.

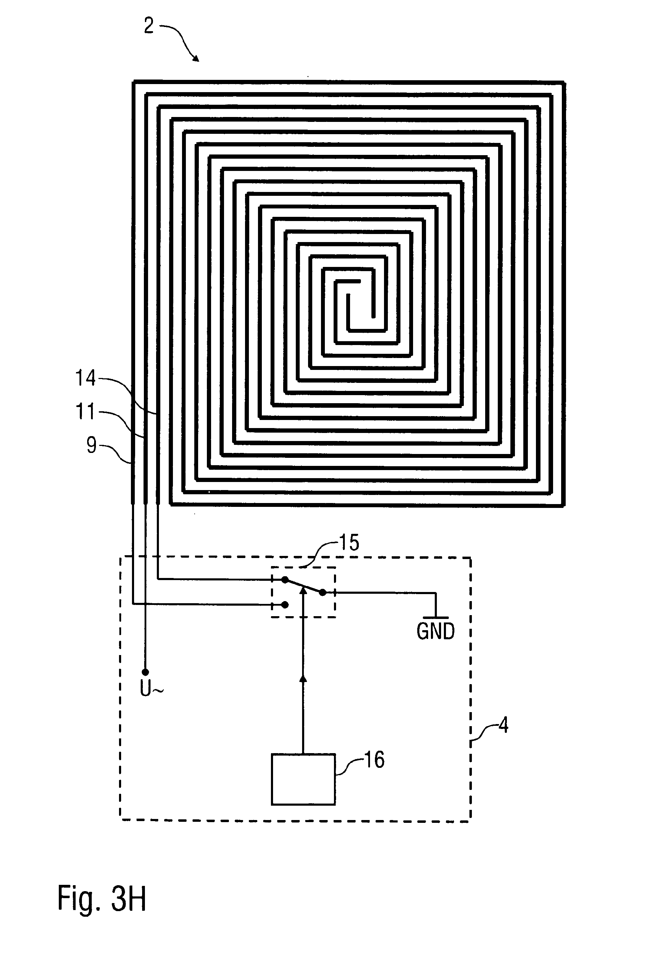

Figure 3H shows a modification of the electrode arrangement 2 according to Figure 3E, so that reference is made to the above description and the same reference numerals are used for corresponding parts and details.

One characteristic of this embodiment is that the electrode arrangement 2 comprises an additional electrode 14 besides the electrodes 9, 11. All the electrodes 9, 11, 14 are spiral-shaped intertwining each other.

Further, the driver circuit 4 comprises one switching element 15 connecting the electrodes 9 and 14 alternatively with ground GND, wherein the switching element 15 is controlled by a control device 16.

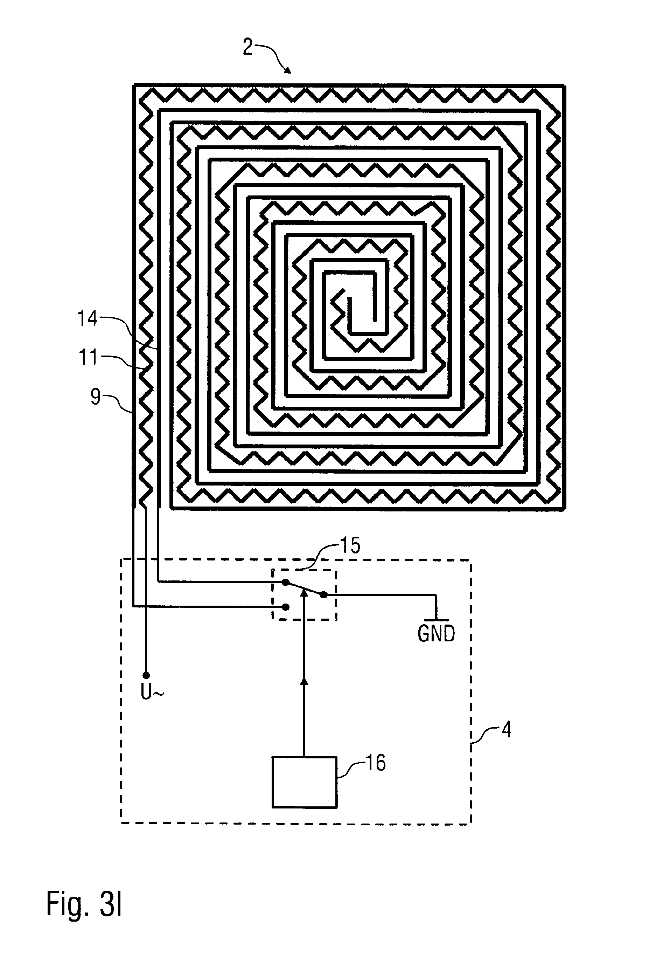

Figure 3I shows a modification of the embodiment of Figure 3H, wherein this embodiment corresponds to the previous embodiment to a large extent. Therefore, reference is made to the above description and the same reference numerals are used for corresponding parts and details.

One characteristic of this embodiment is that the spiral-shaped electrode 11 is staggered.

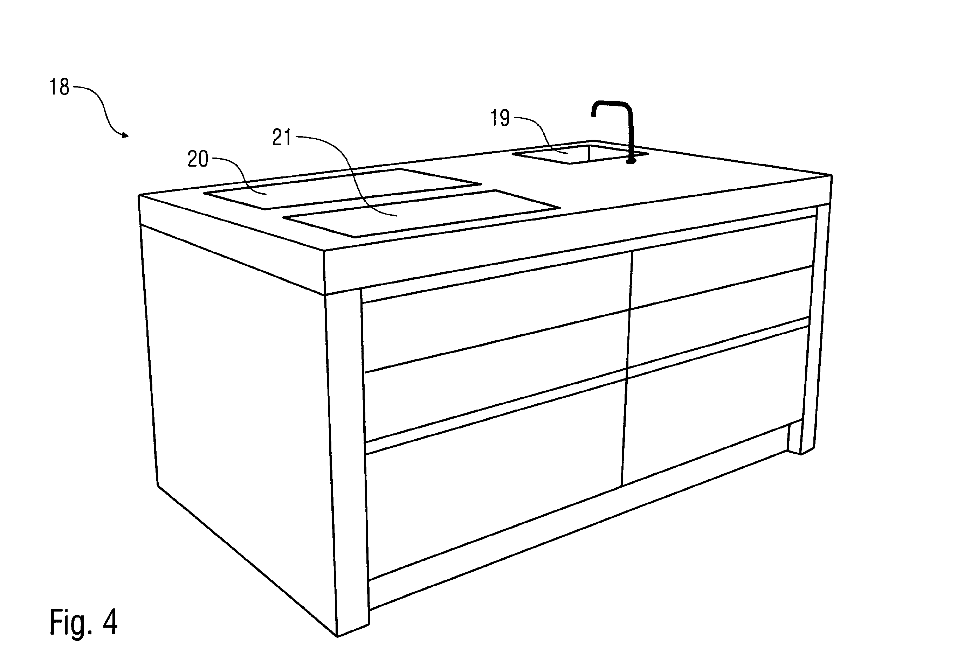

Figure 4 shows a perspective view of a kitchen block 18 comprising a sink 19 and a ceramic stove top 20 which is per se known from the state of the art. However, the kitchen block 18 additionally comprises a self sterilizing work plate 21 comprising an integrated plasma source as mentioned above. The integrated plasma source generates a low-temperature plasma on the surface of the self-sterilizing work plate 21 thereby sterilizing the surface of the work plate 21 at least partially.

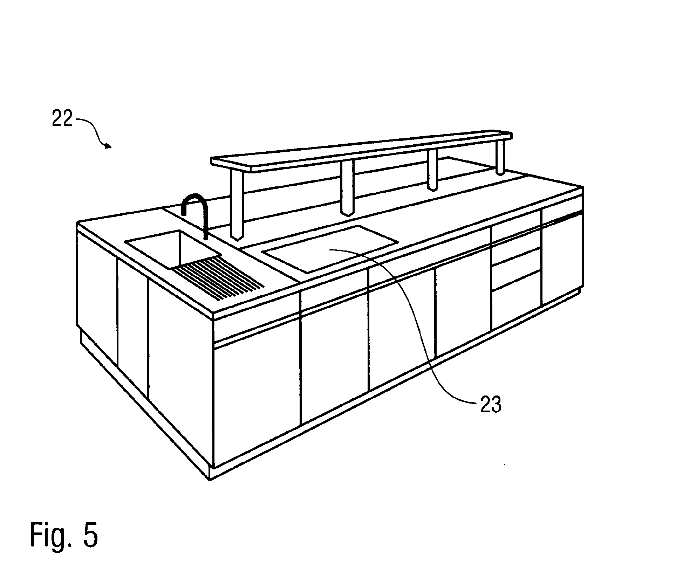

Figure 5 shows a perspective view of a laboratory table 22 comprising a self-sterilizing work plate 23 similar to the self-sterilizing work plate 21 of the kitchen block 18 according to Figure 4.

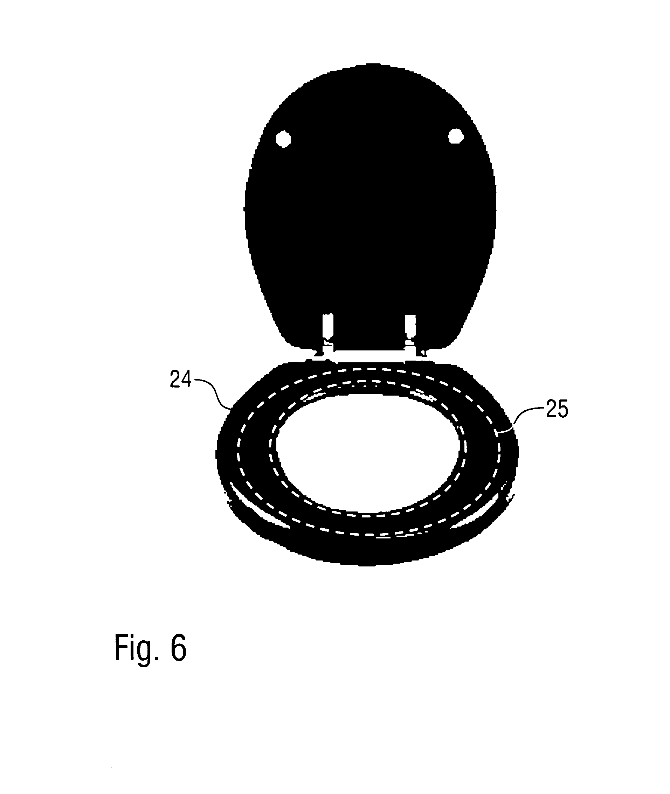

Figure 6 shows a perspective view of a toilet seat 24 comprising an integrated plasma source 25 which is embedded into the toilet seat 24 and generates a low-temperature plasma on the surface of the toilet seat 24 thereby sterilizing the toilet seat at least partially.

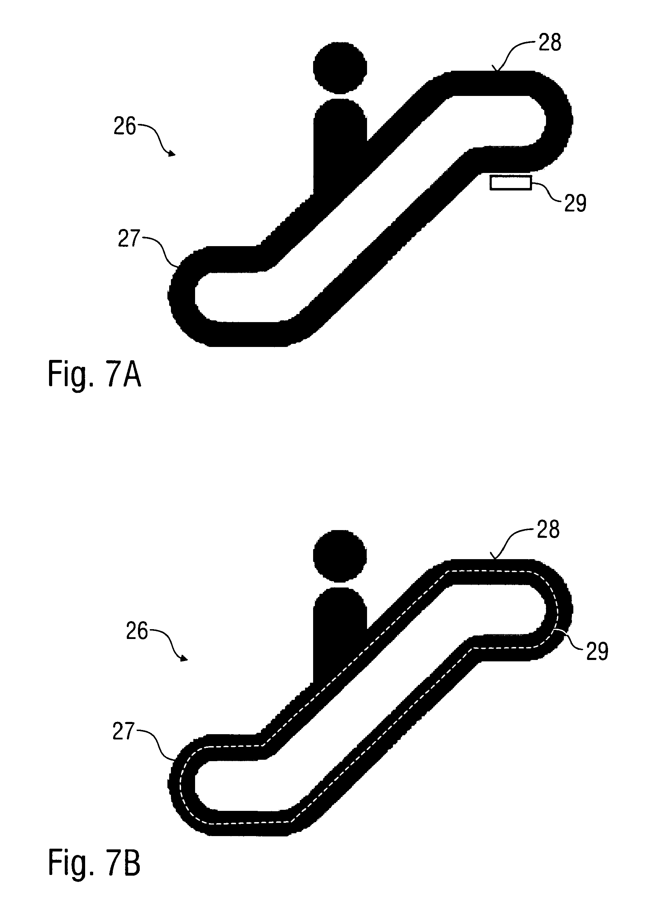

Figure 7A shows a schematic view of an escalator 26 comprising moving handrails 27, wherein the surface 28 of the moving handrails 27 is sterilized by a stationary plasma source 29 which is arranged beneath the moving handrail 27. The plasma source 29 applies a low-temperature plasma to the surface 28 of the moving handrail 27 thereby sterilizing the surface 28.

Figure 7B shows a modification of the embodiment of Figure 7A, wherein this embodiment corresponds to the previous embodiments to a large extent. Therefore, reference is made to the above description and the same reference numerals are used for corresponding parts and details.

One characteristic of this embodiment is that the plasma source 29 is not stationary but arranged within the moving handrail 27 so that the plasma source 29 moves along with the moving handrail 27.

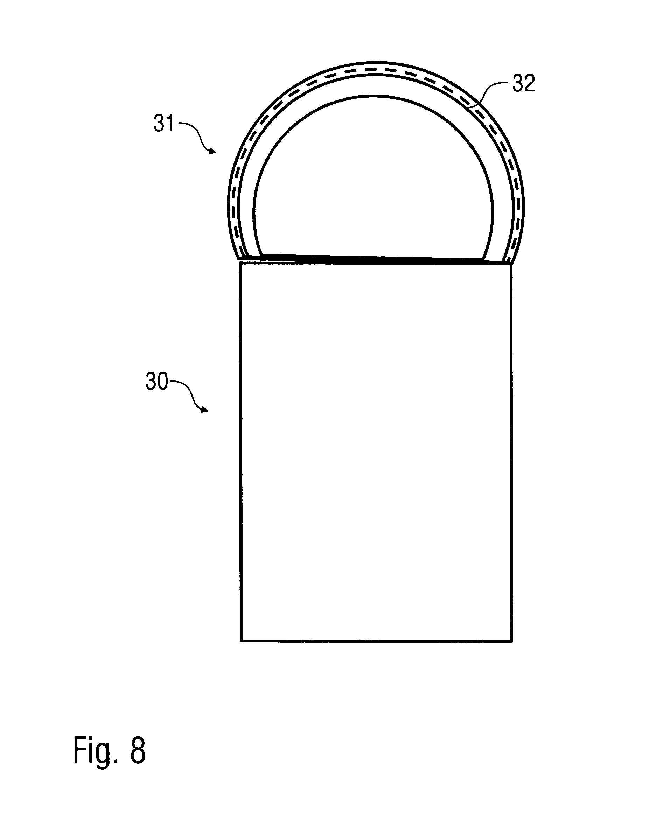

Figure 8 shows a simplified side view of a deodorant device 30 comprising a ball-shaped head 31 with an integrated plasma source 32, wherein the plasma source 32 generates a low-temperature plasma on the surface of the ball-shaped head 31. The deodorant device 30 is used in the same way as conventional deo-rollers, i.e. the ball-shaped head 31 is moved over the body surface to be sterilized so that the low-temperature plasma generated on the surface of the ball-shaped head 31 sterilizes the body surface.

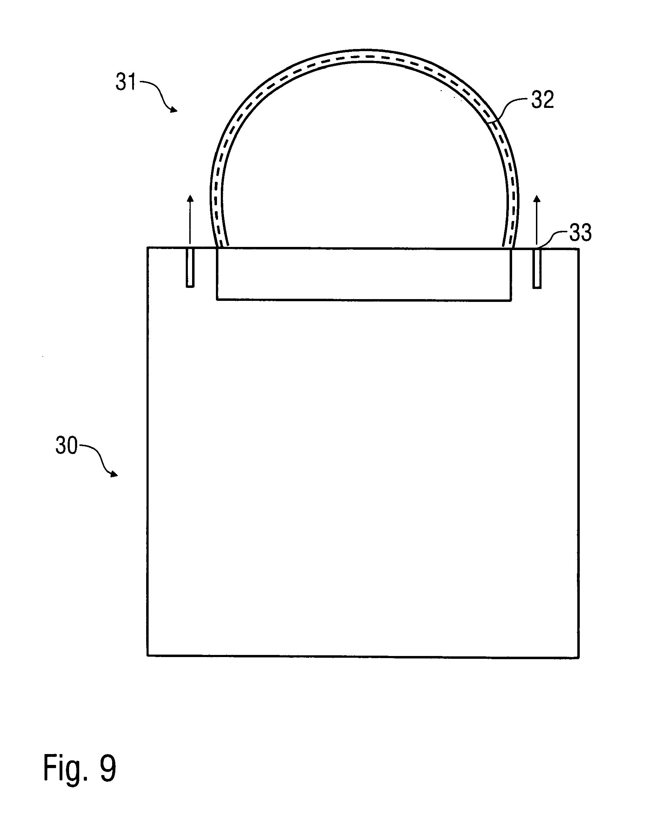

Figure 9 shows a modification of the deodorant device 30 according to Figure 8, wherein this embodiment corresponds to the previous embodiment to a large extent. Therefore, reference is made to the above description and the same reference numerals are used for corresponding parts and details.

One characteristic of this embodiment is that the deodorant device 30 additionally comprises nozzles 33 for applying a chemical agent onto the body surface to be sterilized. The chemical agent applied by the nozzles 33 then interacts with the low-temperature plasma thereby enhancing the sterilizing effect of the low-temperature plasma.

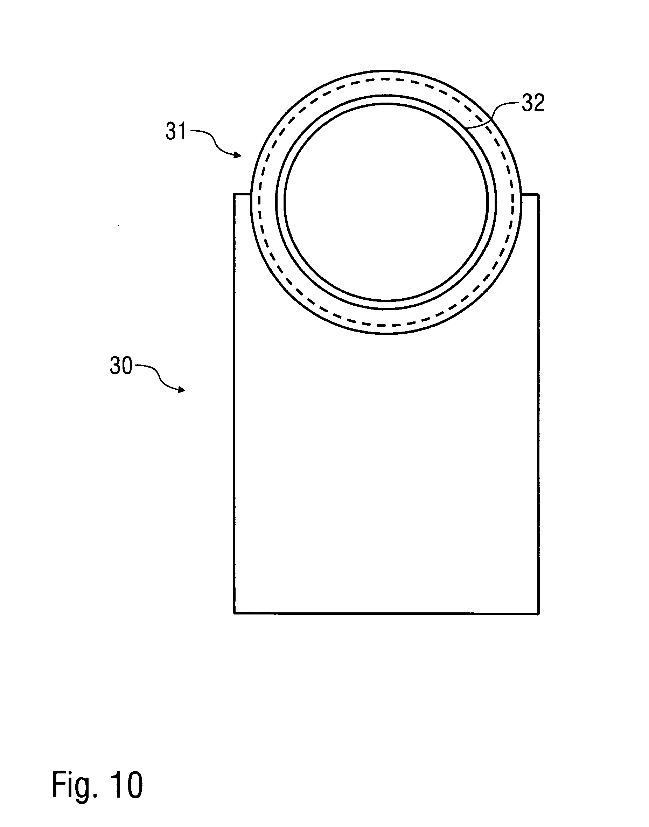

Figure 10 shows a modification of the deodorant device according to Figure 8, wherein this embodiment corresponds to the previous embodiment to a large extent. Therefore, reference is made to the above description and the same reference numerals are used for corresponding parts and details.

One characteristic of this embodiment is that the ball-shaped head 31 is in fact rotatable as in conventional deo rollers.

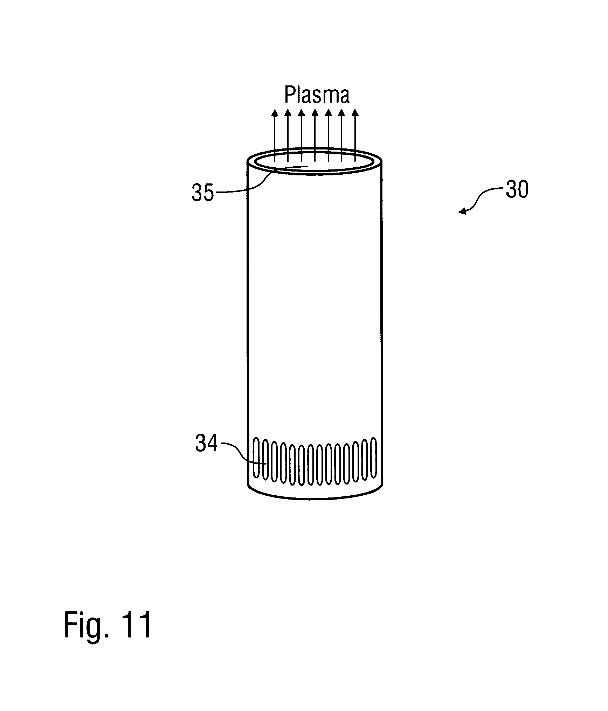

Figure 11 shows another embodiment of a deodorant device 30 including a plasma source in the form of a so-called plasma jet which draws in ambient air through inlet openings 34 at the bottom of the deodorant device 30, while the low-temperature plasma is applied through an outlet opening 35 at the top of the deodorant device 30.

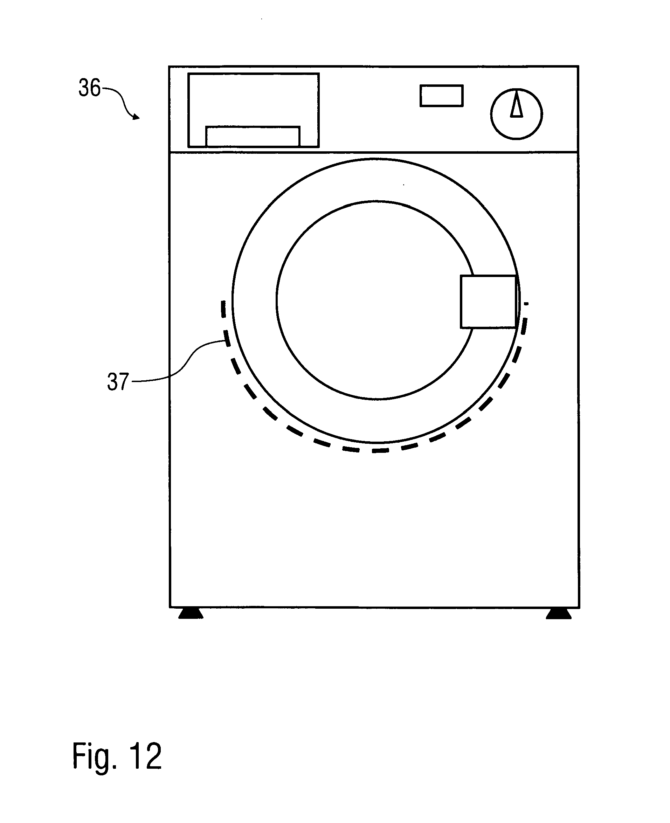

Figure 12 shows a schematic view of a washing machine 36 including a plasma source 37 for applying a non-thermal plasma to the clothes within the washing machine 36.

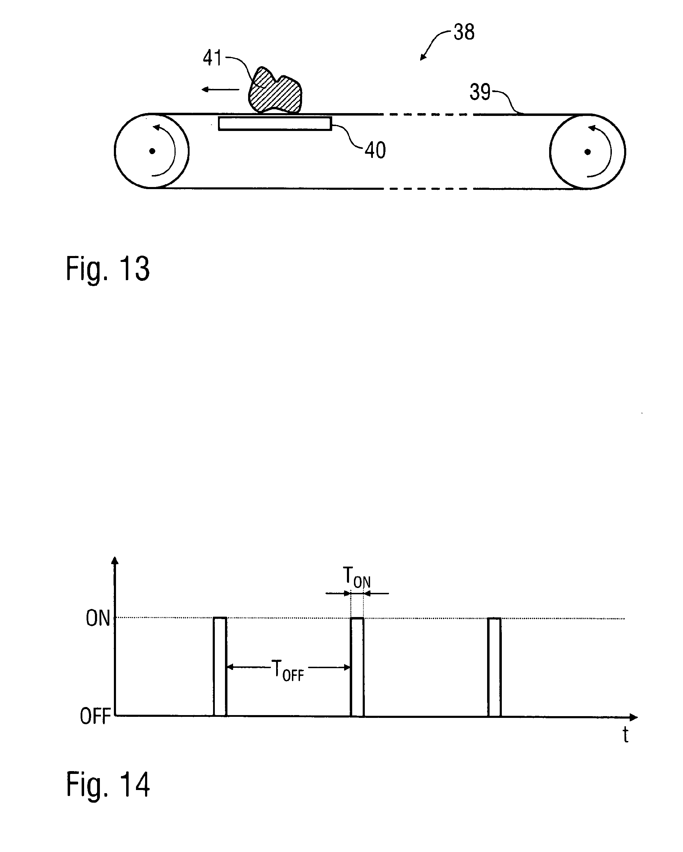

Moreover, Figure 13 shows a schematic side view of a conveyor 38 comprising a conveyor belt 39 and a plasma source 40 which is arranged beneath the upper conveyor belt 39. The plasma source 40 applies a non-thermal plasma through the permeable upper conveyor belt 39 to objects 41 thereby sterilizing the upper surface of the upper conveyor belt 39 and/or the objects 41 on the conveyor belt 39.

Finally, Figure 14 shows a diagram illustrating the on- and off-times of the plasma sources mentioned above. Preferably, the off-time T_{OFF} is much longer than the on-time T_{ON}. In this connection, it should be noted that the sterilization/disinfection also occurs during the off-time T_{OFF} due to the so-called after-glow effect.

Although the invention has been described with reference to the particular arrangement of parts, features and the like, these are not intended to exhaust all possible arrangements of features, and indeed many other modifications and variations will be ascertainable to those of skill in the art.

### List of reference numerals:

- 1: Plasma source
- 2: Electrode arrangement
- 3: Housing
- 4: Driver circuit (high voltage power supply)
- 5: Connection cable
- 6: Appliance
- 7: Surface of the appliance
- 8: Surface of the plasma source
- 9: Electrodes
- 10: Dielectric layer
- 11: Back electrode
- 12: Electrode finger
- 13: Electrode finger
- 14: Electrode
- 15: Switching element
- 16: Control device
- 18: Kitchen block
- 19: Sink
- 20: Ceramic stove top
- 21: Workplate
- 22: Laboratory table
- 23: Workplate
- 24: Toilet seat
- 25: Plasma source
- 26: Escalator
- 27: Moving handrails
- 28: Surface of the handrails
- 29: Plasma source
- 30: Deodorant device

- 31: Ball-shaped head
- 32: Plasma source
- 33: Nozzles
- 34: Inlet openings
- 35: Outlet opening
- 36: Washing machine
- 37: Plasma source
- 38: Conveyor
- 39: Conveyor belt
- 40: Plasma source
- 41: Objects
- GND: Ground

## Claims

1. Appliance (6; 18; 22; 24; 26; 30; 36; 38), particularly kitchen appliance or laboratory table, for at least partially sterilising a contaminated surface (8), **characterized by** an integrated plasma source (1; 25; 32; 37; 40) for at least partially sterilising the surface by generating a non-thermal plasma on the surface thereby reducing the concentration of pathogenic germs on the surface.

2. Appliance (6; 18; 22; 24; 26; 30; 36; 38) according to claim 1, wherein the surface to be sterilised is a surface of the appliance (6; 18; 22; 24; 26; 30; 36; 38) which is contaminated during use of the appliance (6; 18; 22; 24; 26; 30; 36; 38).

3. Appliance (6; 18; 22; 24; 26; 30; 36; 38) according to claim 1 or 2, wherein
a) the plasma source (1; 25; 32; 37; 40) is a surface micro-discharge plasma source (1; 25; 32; 37; 40) comprising several electrodes, and/or
b) there is a uniform distance between the adjacent electrodes of different polarity, or
c) there is a spatially variable distance between the adjacent electrodes of different polarity.

4. Appliance (6; 18; 22; 24; 26; 30; 36; 38) according to claim 3, wherein the surface micro-discharge plasma source (1; 25; 32; 37; 40) is embedded into the surface of the appliance (6; 18; 22; 24; 26; 30; 36; 38), so that the non-thermal plasma is generated on top of the surface of the appliance (6; 18; 22; 24; 26; 30; 36; 38).

5. Appliance (6; 18; 22; 24; 26; 30; 36; 38) according to claim 4, wherein
a) the electrodes of the surface micro-discharge plasma source (1; 25; 32; 37; 40) have a shape which resembles the shape of the surface of the appliance (6; 18; 22; 24; 26; 30; 36; 38), and/or
b) the electrodes of the surface micro-discharge plasma source (1; 25; 32; 37; 40) are flat and/or planar.

6. Appliance (6; 18; 22; 24; 26; 30; 36; 38) according to any of claims 3 to 5, wherein the electrodes of the surface micro-discharge plasma source (1; 25; 32; 37; 40) are arranged in the same plane.

7. Appliance (6; 18; 22; 24; 26; 30; 36; 38) according to claim 6, wherein
a) the electrodes (9, 11) are finger-shaped intertwining each other from opposite directions, or
b) the electrodes (9, 11) are spiral-shaped intertwining each other, and/or
c) the electrodes (9, 11) comprise interlocking branches or kinks.

8. Appliance (6; 18; 22; 24; 26; 30; 36; 38) according to any of claims 3 to 7, wherein
a) the electrically opposite electrodes (9, 11) of the surface micro-discharge plasma source (1; 25; 32; 37; 40) are arranged in separate adjacent electrode layers, and/or
b) each of the electrode layers is planar and the separate electrode layers are arranged coplanar relative to each other, and/or
c) the electrodes are switched at a constant or variable frequency.

9. Appliance (6; 18; 22; 24; 26; 38) according to any of the preceding claims, wherein
a) the surface to be sterilised is substantially plane, and/or
b) the plasma source (1; 25; 37; 40) comprises a substantially plane surface which is flush with the surface which is to be sterilised.

10. Appliance (6; 18; 22; 24; 26; 30; 36; 38) according to any of the preceding claims, wherein the appliance (6; 18; 22; 24; 26; 30; 38) is
a) water-proof,
b) dust-proof,
c) air-borne particles proof, and/or
d) easy to clean.

11. Appliance (6; 18; 22; 24; 26; 30; 36; 38) according to any of claims 3 to 10, wherein the surface consists of a corrosion resistant material, particularly ceramics, glass or glass-ceramics.

12. Appliance (6; 18; 22; 24; 26; 30; 36; 38) according to any of the preceding claims, wherein the appliance (6; 18; 22; 24; 26; 30; 36; 38) is
a) a work plate (21; 23), particularly on a kitchen table (18) or on a laboratory table (22), or
b) a cutting board for cutting objects, particularly foodstuffs, or
c) a handle, particularly a door handle, or
d) bathroom equipment, particularly a toilet seat (24), or
e) a deodorant device (30) for deodorizing a body surface, particularly in the form of a roll-on applicator or a spray device, or
f) a moving handrail (27) of an escalator or a moving walkway, or
g) gym equipment, particularly a bench or a seat of a training machine, or
h) a rechargeable device for mobile sterilisation of surfaces, or
i) a battery operated device for reducing itching caused by insect bites, particularly in the form of a stick comprising the plasma source (1; 25; 32; 37; 40), or
j) suitable for protection against athletes' foot and other fungal diseases, particularly in damp environments, particularly swimming pools and saunas, or
k) suitable for disinfection of baby bottles, pacifier, toys, dentures, tooth brushes, hair brushes, or
l) a dishwasher or a dryer or
m) a conveyor comprising a conveyor belt, wherein the plasma source (1; 25; 32; 37; 40) is arranged in the vicinity of the conveyor belt so that the plasma sterilises objects which are conveyed on the conveyor belt, or
n) a washing machine (36), or
o) a device for disinfecting hands.

13. Appliance (26) according to claim 12 in the form of a moving handrail (27) of an escalator (26) or a moving walkway, wherein
a) the plasma source (29) is integrated into the moving handrail (27), so that the plasma source (27) moves with the handrail (27), or
b) the plasma source (29) is stationary and arranged close to the surface of the handrail (27), so that the non-thermal plasma generated by the plasma source (29) at least partially disinfects or sterilises the surface (28) of the handrail (27).

14. Deodorant device (30) according to claim 12, comprising a rotatable ball (31) for applying a deodorant, wherein
a) the plasma source (32) is integrated into the rotatable ball (31), or
b) the plasma source (32) is arranged stationary outside the rotatable ball (31).

15. Deodorant device (30) according to claim 12 or 14, further comprising an applicator (33) for applying a deodorant comprising at least one nozzle for spraying an agent onto the surface, wherein the agent interacts with the non-thermal plasma thereby improving the disinfecting or sterilising effect of the non-thermal plasma.

16. Deodorant device (30) according to claim 14 or 15, wherein the deodorant device (30) does not comprise any movable parts and relies solely on the disinfecting or sterilising effect of the plasma.

17. Household appliance according to claims 12 in the form of a dishwasher or a dryer, wherein the plasma source is integrated in the walls of the housing of the household appliance.

18. Appliance (6; 18; 22; 24; 26; 30; 36; 38) according to any of the preceding claims, wherein the pathogenic germs comprise
a) bacteria,
b) spores,
c) viruses,
d) fungi,
e) prions,
f) biofilms comprising any of the afore-mentioned pathogenic germs, and/or
g) microorganisms.
